(19) Europäisches Patentamt
European Patent Office
Office européen des brevets

(11) **EP 2 288 329 B1**

(12) **EUROPEAN PATENT SPECIFICATION**

(45) Date of publication and mention
of the grant of the patent:
**01.03.2017 Bulletin 2017/09**

(21) Application number: **09734368.5**

(22) Date of filing: **24.04.2009**

(51) Int Cl.:
*A61K 8/34* (2006.01)     *A61K 8/36* (2006.01)
*A61K 8/21* (2006.01)     *A61K 8/19* (2006.01)
*A61K 8/69* (2006.01)     *A61Q 11/00* (2006.01)
*A61K 8/41* (2006.01)

(86) International application number:
**PCT/EP2009/054993**

(87) International publication number:
**WO 2009/130319 (29.10.2009 Gazette 2009/44)**

(54) **ORAL CARE COMPOSITION COMPRISING DISSOLVED TIN AND FLUORIDE**

MUNDPFLEGEZUSAMMENSETZUNG MIT AUFGELÖSTEM ZINN UND FLUORID

COMPOSITION DE SOINS ORAUX COMPRENANT DE L'ÉTAIN DISSOUT ET DU FLUORURE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **24.04.2008 EP 08155126
26.02.2009 EP 09153802**

(43) Date of publication of application:
**02.03.2011 Bulletin 2011/09**

(73) Proprietor: **GABA International Holding AG
4106 Therwil (CH)**

(72) Inventors:
• **MOYA ARGILAGOS, Dally
CH-8006 Zürich (CH)**
• **MATUR, Turan
CH-4103 Bottmingen (CH)**

• **SCHEFFEL, Cornelia
CH-4147 Aesch (CH)**

(74) Representative: **Jenkins, Peter David
Page White & Farrer
Bedford House
John Street
London WC1N 2BF (GB)**

(56) References cited:
EP-A- 0 026 539        WO-A-94/27565
WO-A-98/22427         US-A- 3 914 404
US-A- 4 828 822        US-A- 5 004 597
US-A- 5 395 241        US-A1- 2003 053 963
US-A1- 2007 025 928

Remarks:
The file contains technical information submitted after
the application was filed and not included in this
specification

**Description**

This application claims the priorities of European patent application no.'s 08155126 and 09153802.

Field of the invention

The present invention relates to the field of oral care compositions, in particular mouthrinses, and their use in treating or preventing erosive tooth demineralization in acidic media, brought about by food acids or endogeneous acids such as gastric juice.

There are three major sources for acids, which can cause tooth demineralization. The first source are the acids generated by cariogenic oral bacteria from food debris. These acids are carboxylic acids derived from the carbohydrates of the food debris that are metabolized by the oral bacteria. Such acids are rather weak, but act for extended periods on the teeth. The second source are the exogeneous food acids that are present in the foodstuffs themselves, in particular in fruits, fruit juices or in artificial softdrinks, or in salad dressings. The third source are endogeneous acids, in particular hydrochloric acid-containing gastric juice, which may come into contact with the teeth upon vomiting, such as in bulimia patients, or in reflux disease patients. These latter two types of acids are rather strong but act only for short times on the teeth. Tooth demineralisation caused by the latter two types of acids is termed "erosive tooth demineralisation" and is not related to cariogenic oral bacteria. Since acid-containing softdrinks have enjoyed a rising popularity among consumers in the past time the problem of erosive tooth demineralisation by food acids has become more acute, and a marked percentage of the overall population is nowadays afflicted by it. Similarly, a rising number of (mainly female) patients are subject to bulimia. Erosive tooth demineralisation is believed to be largely irreversible and is not noticed by the afflicted subject for quite a long time. The pathological condition is thus often only diagnosed at a late stage, when it is already beyond treatment or cure.

Prior art

It has been known for a long time that fluoride ion, such as in the form of sodium fluoride, stannous fluoride, sodium hexafluorophosphate or amine fluoride, is beneficial in preventing tooth demineralisation. These fluorides are customarily administered in the form of oral care products such as toothpastes, dental gels or mouthrinses. In view of the toxicity of fluoride at higher levels the total fluoride concentration in oral care products is kept below a typical level of 1500 ppm.

The applicant of the present application marketed at the time of filing of the priority application to this application a mouthrinse (meridol®), containing the amine hydrofluoride OLAFLUR in an amount corresponding to 125 ppm fluoride and stannous fluoride in an amount also corresponding to 125 ppm fluoride.

It is known that a concentrated aqueous solution (10% by weight) of stannous fluoride, when it acts for prolonged time in vitro on dental enamel, produces an insoluble precipitate on the enamel found to be $Sn_3F_3PO_4$ (Archs. Oral Biol. 16, p. 241ff, 1971).

In a Ph.D. thesis by Anne Schurmann "Effekte unter-schiedlich dosierter lokaler Fluoridapplikationen auf die erosive Demineralisation von humanem Dentin in situ" at the Justus-Liebig-Universität in Giessen, Germany (2004) three oral care products marketed by the applicant of the instant application were tested for their efficacy against erosive tooth demineralisation by citric acid, namely a) meridol® toothpaste, containing OLAFLUR in an amount corresponding to 350 ppm fluoride and stannous fluoride in an amount corresponding to 1050 ppm fluoride; b) the above mentioned meridol® mouthrinse, and c) elmex® gelee containing OLAFLUR in an amount corresponding to 2500 ppm fluoride and sodium fluoride in an amount corresponding to 10000 ppm fluoride, but no stannous salts. Tested were the toothpaste a), the double combination a)+b) and the triple combination a)+b)+c). It was observed that the efficacy against erosive tooth demineralisation increased from a) to double combination a)+b) to triple combination a)+b)+c), which was attributed to the increasing amounts of administered fluoride.

US 5,004,597 A discloses in its examples oral care compositions comprising more than 1000 ppm stannous ions, fluoride and about 10 % by weight of glycerol. The compositions of this publication were devoid of amine fluoride and were not intended for treating or preventing erosive tooth demineralisation.

None of the above mentioned printed publications examined the long-term storage behaviour of the disclosed solutions.

In EP 0 026 539 A it was observed that amine hydrofluorides, when formulated together with stannous fluoride in oral care compositions such as a mouthrinse, stabilizes stannous ions against oxidation and precipitation. Some of its examples were oral care formulations with more than 1000 ppm stannous ion. This publication did, however, not examine the effects of its compositions on erosive tooth demineralisation.

In J. Dent. Res. 50/3, p. 531ff (1971) the efficacy of 0.01 M stannous fluoride solutions (corresponding to about 1180 ppm stannous ion) against erosive enamel demineralisation caused by acetic acid / acetate buffer of pH 4.0 was tested, also after aging up to 21 days, and also in presence of the complexing agents glycerol or tartaric acid. It was found that after 21 days storage the stannous fluoride solution containing one of these two complexing agents was less active in preventing erosive enamel demineralisation than the solution containing stannous fluoride alone.

In a recent publication (Caries Res. 42, pp. 2-7, 2008) stannous chloride solution (815 ppm total tin content) and stannous fluoride solution (809 ppm total tin content) were tested in vitro for the treatment of erosive tooth demineralisation by citric acid. After citric acid erosion and subsequent stannous fluoride treatment the teeth samples seemed even more mineralised than before the erosion test (see its figure 1). The assay for erosion was, however, by X-ray measurements; the absorption reduction caused by tooth demineralisation was compensated partially, or even overcompensated, by the intense absorption of traces of stannous salts deposited onto teeth, thus causing an error in the apparent remineralisation efficacies. Furthermore, since this study was in vitro, it did not consider the influence of the salivary pellicle present in vivo on the teeth on the efficacy of the test solutions analysed.

The present application seeks to provide novel oral care compositions with improved efficacy in the treatment or prevention of erosive tooth demineralisation caused by food acids or endogeneous acids such as gastric juice and which is stable upon prolonged storage.

Summary of the invention

The object set is achieved by an oral care composition comprising a liquid phase containing 30% to 90%, preferably 30% to 80% by weight, based on the liquid phase, of water; dissolved tin; 200 to 2000 ppm fluoride ions, based on the oral composition; and 5 to 60% by weight, based on the oral care composition, of a $C_{(3-5)}$ sugar alcohol; characterised in that the content of dissolved tin [Sn] in the liquid phase is at least 750 ppm, preferably at least 1000 ppm, based on the composition; that 60 mol% or more, preferably 75 mol% or more of the content of dissolved tin [Sn] is in the formal oxidation state +II; and that the composition comprises an organic acid and ammonium cations of the formula (I):

$$R-NH^+R_a-[(CH_2)_u-NH^+R_b]_v-R_c \qquad (I)$$

wherein R is saturated or insaturated straight-chain carbon residue of 10 to 20 carbon atoms, v is an integer from 0 to 1, u is an integer from 2 to 3 and $R_a$, $R_b$ and $R_c$ are independently selected from hydrogen and $-CH_2CH_2OH$; wherein the organic acid is a carboxylic acid, the content of organic acid is 0.01 to 10% by weight, based on the composition and the content of ammonium cations of formula (I) is in the range of 1500 ppm to 10000 ppm, based on the composition, wherein [F⁻] is in the range 0.70 [Sn] $\geq$ [F⁻] $\geq$ 0.40 [Sn], and wherein the composition is a mouthrinse. Preferred embodiments of the oral care composition are as in the dependent claims.

Detained description of the invention

The oral care compositions of the invention contain a liquid phase. By "liquid" is understood in the context of the present application that the phase designated as liquid should have a dynamic viscosity at room temperature of not more than 1000 mPa ·s. The liquid phase is preferably at least partially aqueous. Accordingly, the liquid phase may preferably comprise about 30% to about 90%, more preferably about 30% to about 80% by weight, based on the liquid phase, of water. A possible co-solvent is ethanol, in amounts of typically 5% to 15% by weight, based on the liquid phase.

The term "dissolved tin" is intended to encompass all ionic or non-ionic tin species in the formal oxidation states +II and/or +IV and being dissolved in the liquid phase. Examples of such dissolved tin species are hydrated stannous ions, stannous hydroxide, soluble ionic or nonionic complexes of stannous and/or stannic ions with the dissolved $C_{(3-5)}$ sugar alcohol and/or the anionic conjugate base of the dis solved organic acid as ligands, and ionic hydroxo complexes of stannous and/or stannic ions. In the present mouthrinse composition, 60 mol% or more, more preferably 75 mol% or more of the content of dissolved tin [Sn] is tin in the formal oxidation state +II.

The term "$C_{(3-5)}$ sugar alcohol" is intended to encompass all polyhydric alcohols with a total carbon atom number n of 3 to 5 and a molecular formula of $C_nH_{(2n+2)}O_n$. Preferably these sugar alcohols are acyclic and unbranched. Examples of the $C_{(3-5)}$ sugar alcohol are glycerol, erythritol, threitol, arabitol, xylitol and ribitol. More preferred are acyclic unbranched $C_{(3-4)}$ sugar alcohols, such as glycerol, erythritol and threitol, and particularly preferred is glycerol. A more preferred range of content for the dissolved $C_{(3-5)}$ sugar alcohol is 25% to 45% by weight, based on the oral care composition. The $C_{(3-5)}$ sugar alcohol is preferably dissolved in the liquid phase.

The organic acid is a carboxylic acid. It is preferably dissolved in the liquid phase of the composition. The term "dissolved" implies here that the acid be dissolved either as the free acid or as a pharmaceutically acceptable salt of its anionic conjugate base (whichever may be the case) in the liquid phase of physiologically acceptable pH. Preferred subgroups of organic acids are edible di- or tricarboxylic acids with 4 to 6 carbon atoms including the carboxylate carbon atoms, such as succinic, tartaric, citric, malic, fumaric and adipic acids; or edible $\alpha$-hydroxy $C_{(2-6)}$carboxylic acids such

as glycolic, lactic, citric, tartaric or gluconic acids. If the organic acid is dissolved in the form of a pharmaceutically acceptable salt then the counter cation may be a metal cation, such as from an alkaline metal (such as sodium or potassium), from an earth alkaline metal (such as magnesium or calcium), or from zinc. As an alternative the counter cation may be an ammonium cation of the above formula (I).

The content of the organic acid is in the range of 0.01 to 10% by weight, preferably 0.05 to 3% by weight, based on the composition, whereby the upper limit may be given by the solubility of its conjugate base salt in the liquid phase at physiologically acceptable pH. The total content of organic acids may be determined by acidifying a known aliquot of the oral care composition to about pH 0, extracting the free organic acids with an organic solvent such as ether, and analysing the extract by calibrated GC using the silyl esters derivates of the acids.

More preferably the content of dissolved tin [Sn] in the liquid phase is in the range of 1000 ppm to 3000 ppm, even more preferably in the range of 1300 ppm to 2500 ppm, still even more preferably in the range of 1700 ppm to 2200 ppm, and most preferably in the range of 1900 to 2100 ppm, based on the composition. The total content of dissolved tin may be determined using X-ray fluorescence (see example 13). The content of dissolved tin in the formal oxidation state +II may be determined potentiometrically (see example 14). The dissolved tin may preferably be derived from a pharmaceutically acceptable stannic ion salt added to the oral care formulation. Examples are stannous chloride, stannous fluoride, stannous hydroxide, stannous sulfate, with stannous chloride being preferred.

The combination of dissolved tin species, of which some are rather acidic, and the pharmaceutically acceptable salt of the organic acid, which is rather basic, may yield in the liquid phase of the oral composition a pH range which is physiologically acceptable for an oral care composition, such as typically about 3.0 to about 7.0, preferably about 4.0 to about 5.0, more preferably about 4.3 to about 4.6. if necessary the pH of the oral care composition may be adjusted to the desired value by adding acid (such as hydrochloric acid) or base (such as sodium hydroxide).

The fluoride content of the oral care compositions is from 200 to 2000 ppm, based on the composition, preferably from 500 to 1000 ppm. Preferably the fluoride is dissolved in the liquid phase of the composition. The fluoride content of the oral care composition may be determined potentiometrically using a fluoride-selective electrode (see example 15). The fluoride may be added to the oral care composition in the form of any fluoride ion source customarily employed in oral care compositions, e.g. as stannous fluoride and/or sodium fluoride and/or as above mentioned amine fluoride.

The content of fluoride ions in ppm, based on the composition, $[F^-]$, is in the range of $0.70[Sn] \geq [F^-] \geq 0.40[Sn]$, more preferably $0.60[Sn]$ $[F^-] \geq 0.40[Sn]$, wherein [Sn] has the above meaning. In another preferred embodiment the content of fluoride ions in ppm, based on the composition, $[F^-]$, is in the range of $0.30[Sn] \geq [F^-] \geq 0.20[Sn]$, provided that the content of dissolved tin, [Sn], as described above, is then in the range of 1900 to 2200 ppm or in the range of 1000 to 1400 ppm, preferably 1050 to 1250 ppm, based on the composition. In all these preferred embodiments, preferably 80 mol% or more, or even 90 mol% or more, of the content of dissolved tin [Sn] is in the formal oxidation state +II. In all these preferred embodiments, the composition is preferably a mouthrinse.

The ammonium cations of formula (I) are derived from corresponding amines which contain one or two basic nitrogen atoms and which are converted to the ammonium cations of formula (I) by adding an amount of an acid which is acceptable in an oral care composition, such as hydrochloric, hydrofluoric, carbonic, citric, lactic or gluconic acids, preferably hydrochloric or hydrofluoric acids, and most preferably hydrofluoric acid, in which latter case the acid addition salts are known as "amine hydrofluorides" or "amine fluorides". The content of ammonium cations is in the range of 1500 ppm to 10000 ppm, based on the composition. Preferably the ammonium cations of formula (I) are dissolved in the liquid phase of the composition. The determination of the content of ammonium cations of formula (I) may be done over their corresponding free amine bases using calibrated reverse phase HPLTC (see examples 16 and 17).

In the ammonium cations of formula (I) the residue R can have even or odd-numbered chain length. Preferably the carbon atom of R which is connected to the nitrogen atom of formula (I) forms a methylene group. Residues R having an even-numbered chain length are preferred with regard to physiological acceptability. The residues may be saturated or mono-, di- or polyunsaturated, preferably mono-unsaturated. Examples of saturated hydrocarbon residues having an even-numbered chain length are decyl, dodecyl (lauryl), tetradecyl (myristyl), hexadecyl (cetyl, palmityl), octadecyl (stearyl) and eicosanyl. Examples of unsaturated residues having an even-numbered chain length are 9-cis-octadecen-1-yl (oleyl), 9-trans-octadecen-1-yl (elaidyl), cis,cis-9,12-octadecadien-1-yl (linolyl), cis,cis,cis-9,12,15-octadecatrien-1-yl (linolenyl) or 9-cis-eicosaen-1-yl (gadolyl). Preferred are even-numbered $C_{(16-20)}$alkyl or even-numbered $C_{(16-20)}$alkenyl. More preferred are those cations of formula (I) wherein R is 9-trans-octadecen-1-yl (elaidyl), cis,cis-9,12-octadecadien-1-yl (linolyl), cis,cis,cis-9,12,15-octadecatrien-1-yl (linolenyl) or 9-cis-eicosaen-1-yl (gadolyl). Preferred are even-numbered $C_{(16-20)}$alkyl or even-numbered $C_{(16-20)}$alkenyl. More preferred are those cations of formula (I) wherein R is $C_{18}$alkyl or $C_{18}$alkenyl, and most preferred wherein R is 9-cis-octadecen-1-yl (oleyl).

The acid addition salts mentioned above containing the ammonium cations of formula (I) may be prepared in all instances by reacting the corresponding free amine base $R-NR_a-[(CH_2)_u-NR_b]_v-R_c$, wherein all symbols have the same meaning as in claim 1, with the appropriate acid in one equivalent, or slightly more than one equivalent (such as 1.05 equivalent) hydronium per basic nitrogen atom present in the free amine base. If the free amine base is a pure compound, the number of basic nitrogen atoms is clear from the structural formula. If the amine base is, however, a mixture of compounds, then the number of basic nitrogen atoms may be determined by titration of a sample of such a mixture with perchloric acid in glacial acetic acid using a glass electrode.

The preparation of the free amine bases themselves is briefly described in the following sections i) to iii).

i) In the case where v is 0 and $R_a$, $R_c$ are hydrogen the amine base is simply a fatty amine R-NH$_2$, wherein R has the meaning of claim 1.

ii) In the case where v is 0 and at least one of $R_a$ and $R_c$ is -CH$_2$CH$_2$OH the amine may be obtained by hydroxyethylation of a fatty amine R-NH$_2$, with R as defined in claim 1, with one equivalent ethylene oxide, which gives the amines with $R_a$ as H and $R_c$ as -CH$_2$CH$_2$OH; or with two equivalents of ethylene oxide, which gives the amines with $R_a$, $R_c$ as -CH$_2$CH$_2$OH.

iii) In the case where v is 1 and u is 2 or 3 they may be prepared by acylation of ethylene diamine or propylene diamine, respectively, with an acyl chloride R'-COCl, which gives firstly amides R'-CO-NR$_a$-[(CH$_2$)$_u$-NR$_b$]$_v$-R$_c$ with $R_a$, $R_b$ and $R_c$ as hydrogen. Reaction of the non-acylated nitrogen atom of these with one equivalent ethylene oxide gives the corresponding amides with $R_a$, $R_b$ as hydrogen and $R_c$ as -CH$_2$CH$_2$OH; or with two equivalents of ethylene oxide the corresponding amides with $R_a$ as hydrogen and $R_b$, $R_c$ as -CH$_2$CH$_2$OH. To furthermore introduce $R_a$ as -CH$_2$CH$_2$OH any of these amides may be reacted at the amide nitrogen (the one having $R_a$ connected to it) with bromoethanol in the presence of a strong base such as t-BuOK, optionally with beforehand protection of the hydroxyl groups with dihydropyran. Any of the amides so obtained may then be reduced to the corresponding amine with lithium aluminium hydride, optionally with beforehand protection of any present hydroxyls with dihydropyran, to obtain amines R'-CH$_2$-NR$_a$-[(CH$_2$)$_u$-NR$_b$]$_v$-R$_c$, wherein R'-CH$_2$ is now equal to R of claim 1. If after the acylation step the said reaction sequence is inverted (i.e. first alkylation at the amide nitrogen with 1-bromoethanol/t-BuOK, then reaction with one equivalent of ethylene oxide), and then the reduction with lithium aluminium hydride is performed, then the amine bases with $R_a$, $R_b$ as -CH$_2$CH$_2$OH and $R_c$ as hydrogen are accessible.

As already stated the ammonium cations of formula (I) are most preferably added to the oral care composition as amine hydrofluorides. The amine hydrofluoride where R is 9-octadecen-1-yl (oleyl), v is 0 and $R_a$, $R_c$ are hydrogen is known under the international non-proprietary name of DECTA-FLUR. The amine hydrofluorides where v is 0, $R_a$ and $R_c$ are - CH$_2$CH$_2$OH and R is octadecyl or 9-octadecen-1-yl are known from the examples of WO 98/22427 A. The latter one of these two is known under the international non-proprietary name of XIDECAFLUR. The amine fluoride where v is 1, u is 3, and $R_a$, $R_b$ and $R_c$ are -CH$_2$CH$_2$OH is known under the international non-proprietary name of OLAFLUR. OLAFLUR, DECTAFLUR and XIDE-CAFLUR are the particularly preferred amine hydrofluorides, and most preferred is OLAFLUR.

The oral care compositions may also comprise chloride ions, preferably as dissolved ions in the liquid phase. A preferred range of the chloride content [Cl$^-$] in ppm, based on the composition, is in the range 0.65[Sn] $\geq$ [Cl] $\geq$ 0.55[Sn], preferably in the range 0.62[Sn] $\geq$ [Cl] $\geq$ 0.56[Sn], and most preferably is about 0.60[Sn]. The chloride content may be determined by potentiometric titration (see example 18). The chloride may be added for example as sodium chloride, calcium chloride or stannous chloride, with the latter being preferred.

The oral compositions of the invention are preferably devoid of copper, meaning that they comprise preferably less than 0.05% by weight, more preferably less than 0.001% by weight, based on the composition, of copper.

It is understood that the oral care composition of the present invention is electroneutral, i.e. the sum of the negative charges brought about by all anions present is equal to the sum of all cations present.

The oral care composition of the invention is a mouthrinse.

The compositions of the invention are clear solutions essentially, preferably completely free of suspended or sedimented solids or from turbidity.

The compositions of the invention are efficacious in the treatment or prevention, particularly the prevention of erosive tooth demineralisation caused by food acids (i.e. acids originating from foods) or by endogeneous acids such as gastric juice. As "food acids" are considered in the context of the present application in particular phosphoric, acetic, propionic, benzoic, carbonic, citric, malic, oxalic, lactic, pyruvic, succinic, tartaric, tannic, caffeotannic, ascorbic, gluconic, glucuronic and glucaric acids, pectin, hydrated sulfur dioxide, and amino acids; and any salts thereof still containing at least one hydrogen atom which is dissociable to at least 50 mol% in aqueous solution at a pH typical for human saliva (i.e. a pH about 5.6 to about 8.4). Particularly are understood as food acids such acids with a first pKa value of 3.0 or less (such as phosphoric and citric acids, hydrated sulfur dioxide and aspartic acid), and/or which can act as chelating ligands for calcium ions (such as lactic, tartaric, citric, malic and amino acids) and/or which form low-soluble calcium salts (such as oxalic, carbonic and phosphoric acids). As "low soluble calcium salts" are understood in the present application calcium salts with a solubility of less than 0.1 g / 100 ml water of pH 5.7 at ambient temperature and pressure and at 35 Pa partial pressure of carbon dioxide. "low soluble calcium salts" are understood in the present application calcium salts with a solubility of less than 0.1 g / 100 ml water of pH 5.7 at ambient temperature and pressure and at 35 Pa partial pressure of carbon dioxide.

Further optional components in all types of oral care composition of the invention may be for instance:

- Flavourings and cooling flavours, such as coumarin, vanillin, ethereal oils (such as peppermint oil, spearmint oil, aniseed oil, menthol, anethol or citrus oil) or other essences (such as apple, eucalyptus or spearmint essence). These flavourings may be present in 0% to 0.5%, preferably 0.03% to 0.3% by weight, based on the oral care composition.
- Sweeteners, in particular artificial sweeteners such as saccharin, acesulfam, neotam, cyclamate or sucralose; natural high-intensity sweeteners such as thaumatin, stevioside or glycyrrhizin; or sugar alcohols different from the $C_{(3-5)}$ sugar alcohol, such as sorbitol, xylitol, maltitol or mannitol. These may be present in amounts of 0% to 0.2%, preferably 0.005% to 0.1% by weight, based on the composition.
- Antibacterials and/or preservatives, such as chlorhexidine, triclosan, quaternary ammonium compounds (such as benzalkonium chloride) or parabens (such as methyl or propyl paraben). The amount of antimicrobial agent in the oral care composition is typically from 0 to about 0.5%, preferably 0.05 to 0.1% by weight, based on the oral care composition.
- Emulsifiers or solubilisers, mainly in connection with abovementioned flavourings and/or antibacterials, which often are of low solubility in aqueous media. Examples of such emulsifiers are neutral surfactants (such as polyoxyethylene hydrogenated castor oil or fatty acids of sugars), anionic surfactants (such as sodium lauryl sulfate), cationic surfactants (such as the ammonium cations of formula (I)) or zwitterionic surfactants. These surfactants or solubilisers may be present in amounts of typically 0% to 2%, preferably 0.2% to 1.5% by weight, based on the oral care composition.
- Thixotropic agents, such as soluble grades of hydroxypropylmethylcellulose, hydroxyethylcellulose or mucins, in an amount effective to impart the oral care composition a thixotropic behaviour.
- Stabilisers, such as polyvinylpyrrolidone.

Further optional components for oral care compositions of the invention that have a solid phase, such as in particular toothpastes or dental gels, are abrasives, such as inorganic abrasives (e.g. silica, aluminium oxide, calcium carbonate, calcium phosphate, calcium pyrophosphate or stannous pyrophosphate) or organic abrasives (such as polyethylene, polyvinyl chloride, polystyrene, polycarbonate, copolymers from (meth)acrylates and other olefinic monomers, polyamides, urea-formaldehyde resins, melamine-formaldehyde resins, phenol-formaldehyde resins, cured, pulverised epoxy resins or polyesters).

The oral care compositions of the invention may be used to treat or prevent erosive tooth demineralisation in a subject in need of such treatment or prevention. For this application it is preferred that the oral care composition be a mouthrinse. The mouthrinse is preferably provided to the subject in need in the form of a package containing both the mouthrinse and a leaflet onto which the instruction is printed to use the mouthrinse typically once a day, in an amount of typically 5 to 30 ml, preferably about 10 to 20 ml, depending on its content of the essential four ions, with the instructions to the subject to rinse the oral cavity for a certain period of time which is typically about 10 seconds to 1 minute, preferably about 30 seconds, again depending on the content of the mouthrinse, thus bringing the subject's teeth in contact with the mouthrinse. After the rinsing the mouthrinse may be discarded without swallowing, and preferably no rinsing of the oral cavity with water is performed afterwards. Such an administration regime is similar to the administration regime for mouthrinses of the prior art.

The oral care compositions of the instant invention are efficacious against erosive tooth demineralisation despite the fact that they preferably contain fluoride in an amount relative to the tin content which is neither as in stannous fluoride itself nor as in $Sn_3F_3PO_4$ (see the introduction), such as $0.60[Sn] \geq [F^-] \geq 0.40[Sn]$. The oral care compositions of the invention are stable and thus remain clear and precipitate-free for prolonged storage time. The oral care compositions of the present invention cause no coloration of the teeth, nor irritation of the gums, despite the fact that they contain appreciably higher amounts of tin than the known meridol® mouthrinse marketed by the applicant himself.

The invention will now be further explained by the following non-limiting examples.

Examples 1-12: Mouthrinse formulations

In the following examples "AmF" or "AmF 297" denotes the amine hydrofluoride OLAFLUR. The amounts of all ingredients listed in the table are in percentages by weight, based on the overall mouthrinse. (Examples 1,9-12 are not part of the invention as claimed)

| Example No. | 1 | 2 | 3 | 4 | 5 | 6 |
|---|---|---|---|---|---|---|
| Added actives | 750 ppm F⁻ ex AmF, 750 ppm F⁻ ex NaF, [Sn] 2800 ppm | 500 ppm F⁻ ex AmF, 500 ppm F⁻ ex NaF, [Sn] 2100 ppm | 250 ppm F⁻ ex AmF, 750 ppm F⁻ ex NaF, [Sn] 1400 ppm | 500 ppm F⁻ ex AmF, 500 ppm F⁻ ex NaF, [Sn] 2100 ppm | 250 ppm F⁻ ex AmF, 1000 ppm F⁻ ex NaF, [Sn] 2100 ppm | 150 ppm F⁻ ex AmF, 850 ppm F⁻ ex NaF, [Sn] 2100 ppm |
| AmF solution | 5.357 | 3.571 | 1.786 | 3.571 | 1.786 | 1.072 |
| SnCl$_2$ dihydrate | 0.534 | 0.408 | 0.272 | 0.407 | 0.407 | 0.407 |
| NaF | 0.166 | 0.1105 | 0.166 | 0.1106 | 0.2211 | 0.188 |
| Zinc lactate | | 0.75 | 0.75 | | 1 | 0.75 |
| HCl 20% | | | 0.066 | 0.44 | 0.06 | 0.07 |
| KOH 20% | 0.1 | | | | | |
| Tego betain F50 | | 1 | 1 | 0.8 | 0.8 | 1.4 |
| Cremophor RH 410 | | | | 1 | 0.5 | 0.2 |
| Aroma | | | | 0.44 | 0.22 | 0.14 |
| Sodium saccharin | | | | 0.06 | | 0.05 |
| Acesulfam K | | | | | 0.1 | |
| Glycerol | 30 | 35 | 30 | 30.5 | 26 | 39 |
| Deionised water | 63.843 | 59.1605 | 65.96 | 61.6714 | 68.9059 | 55.967 |
| Sodium lactate | | | | | | 0.75 |
| Sodium citrate | | | | 1 | | |
| Sodium D-gluconate | | | | | | |

| Example No. | 7 | 8 | 9 | 10 | 11 | 12 |
|---|---|---|---|---|---|---|
| Added actives | 150 ppm F⁻ ex AmF, 850 ppm F⁻ ex NaF, [Sn] 2100 ppm | 500 ppm F⁻ ex AmF, 500 ppm F⁻ ex NaF, [Sn] 1900 ppm | 150 ppm F⁻ ex AmF, 350 ppm F⁻ ex NaF, [Sn] 2100 ppm | 125 ppm F⁻ ex AmF, 375 ppm F⁻ ex NaF, [Sn] 2100 ppm | 150 ppm F⁻ ex AmF, 350 ppm F⁻ ex NaF, [Sn] 2100 ppm | 125 ppm F⁻ ex AmF, 375 ppm F⁻ ex NaF, [Sn] 2100 ppm |
| AmF solution | 1.072 | 3.571 | 1.072 | 0.893 | 1.072 | 0.893 |
| SnCl$_2$ dihydrate | 0.403 | 0.364 | 0.407 | 0.403 | 0.405 | 0.405 |
| NaF | 0.188 | 0.1105 | 0.0774 | 0.083 | 0.0774 | 0.083 |

| | | | | | | |
|---|---|---|---|---|---|---|
| Zinc lactate | 0.75 | 0.75 | | 0.5 | | 0.5 |
| HCl 20% | 0.07 | 0.05 | | 0.03 | | 0.03 |
| KOH 20% | | | 0.15 | | 0.21 | |
| Tego betain F50 | 1.4 | 1 | 1 | 0.4 | 0.4 | 0.5 |
| Cremophor RH 410 | 0.2 | 0.3 | 0.25 | 0.2 | 0.2 | 0.45 |
| Aroma | 0.13 | 0.27 | 0.14 | 0.12 | 0.2 | 0.18 |
| Sodium saccharin | 0.055 | 0.1 | 0.05 | 0.035 | 0.12 | 0.0175 |
| Acesulfam K | | | | | | |
| Glycerol | 39 | 26 | 41 | 32 | 28 | 28 |
| Deionised water | 55.987 | 67.4005 | 54.8536 | 63.676 | 68.315 | 67.2915 |
| Sodium lactate | 0.75 | | | 1.5 | | 1.5 |
| Sodium citrate | | | | | | |
| Sodium D-gluconate | | | 1 | 0.15 | 1 | 0.15 |

Example 13: Determination of the total content of dissolved tin [Sn] by X-ray fluorescence in an oral care composition of the invention

As the x-ray fluorescence spectrometer a Thermo Noran QuanX is used. Two solutions are measured:

Solution 1: 5 g of the oral care composition is directly filled into a XRF-cup. The XRF-cup is then closed with a polyethylene foil with the appropriate closing ring and is followingly inserted into the autosampler of the instrument.

Solution 2 is as solution 1, but with a known amount of furthermore added stannous salt [$\Delta$Sn] in the range of 80% to 120% of the expected ppm value of [Sn] of the sample solution.

Solutions 1 and 2 are each irradiated for 600 seconds with x-ray at 50 kV excitation, using a copper filter, $K_\alpha$-line at 25.193 keV. The integrated area under the fluorescence intensity peak of solution 1 is taken as $A_1$ and the integrated area under the fluorescence intensity peak of solution 2 is taken as $A_2$.

The dissolved tin content in ppm based on the composition, [Sn], is obtained as

$$[\text{Sn}] = [\Delta \text{Sn}] \frac{A_2}{A_2 - A_1}$$

Example 14: Measurement of dissolved tin at formal oxidation state +II in an oral care composition of the invention

A combined platinum electrode type 6.1204.310 of Metrohm, Switzerland, and a potentiometer Titrando 809 of Metrohm, Switzerland, are used. The calibration of the electrode is done according to the manual.

10.0000 g of the oral care composition are exactly weighed ($\pm$ 0.1 mg) in a 100 ml container and 40 ml water, 5 ml 32wt% HCl and a known aliquot v (in ml) of standard 0.05 M $KI_3$ solution is added, such that iodine is added in excess of the tin in formal oxidation state +II contained in the sample (a typical value for v is 5 ml).

The electrode is immersed into the sample solution and the remaining iodine not already reduced to I- by the tin in formal oxidation state +II is titrated back with standard 0.1 M $Na_2S_2O_3$ solution to the endpoint of the titration. The used amount of $Na_2S_2O_3$ solution in ml is taken as $v_1$.

The tin in formal oxidation state +II contained in the sample in ppm based on the oral composition, [$Sn^{+II}$], is obtained as

$$[\text{Sn}^{+II}] = 593.45 \ (v - v_1)$$

Example 15: Potentiometric fluoride determination in an oral care composition of the invention

A fluoride-selective electrode type 6.0502.150 of Metrohm, Switzerland, a pH/Ion-meter 692, Metrohm, Switzerland and an Ag/AgCl reference electrode type 6.0750.100, Metrohm, Switzerland are used.

A total ionic strength adjusted buffer (TISAB) is required and made as follows: A solution of 160 mg NaOH in 2 litres of water is prepared (solution 1); 25 g 1,2-diamino-cyclohexane-N,N,N',N'-tetraacetic acid, 290 g NaCl and 285 ml glacial acetic acid are dissolved in 2 litres of water (solution 2); then solutions 1 and 2 are mixed and filled up to 5 litres with water.

The calibration of the fluoride-selective electrode is performed according to the manual of the pH/Ion-meter.

1.0000 g $\pm$ 0.1 mg of the oral care composition are exactly weighed in a 50 ml plastic container and filled up with water to a weight of 20.0000 g $\pm$ 0.1 mg, and 20 ml of above mentioned TISAB buffer are added. The fluoride-selective electrode and the reference electrode are immersed into the sample and the potential is read off after 5 minutes, according to the manual of the pH/Ion-meter. The fluoride concentration in ppm is calculated by multiplying the measured response-value by 40 (the total dilution factor from the oral care composition to the measured sample), and dividing by the weight of the oral care composition sample in g.

Example 16: Determination of ammonium cations of formula (I) with $R_a$, $R_c$ = hydrogen and v = 0, or with $R_b$, $R_c$ = hydrogen and v = 1, in an oral care composition of the invention

The determination is done using densitometric quantification on reverse phase HPTLC plates after staining with ninhydrine.

Procedure:

Ninhydrine solution: Dissolve 2 g of ninhydrine purum in 1000 ml of ethanol p.a. The solution has to be stored in a glass bottle at 4 °C (maximal storage time: 1 month).

A reference solution of the ammonium cation to be determined is prepared by dissolving an exactly known amount of the corresponding pure amine hydrofluoride in methanol p.a., to make a solution containing an exactly known content of the amine fluoride in the range of about 3000 ppm, based on the solution. This reference solution is designated in the following as R.

Sample solution: Accurately weigh (to within 0.1 mg) an amount M of approximately 1 g of the oral care composition in a 25 ml measuring flask and make up to volume with methanol p.a. Expose to ultrasonic radiation for about 20 minutes. This solution is designated as S.

The HPTLC plate is Silicagel 60 without fluorescence indicator, 10 x 20 cm (Merck no. 5626).

The reference solution and the sample solution are applied onto the HPTLC plate using an applicator Linomat IV (Camag, Switzerland) according to the following track scheme:

| Track No. | Solution | Amount applied ($\mu$l) |
| --- | --- | --- |
| 1 | R | 2 |
| 2 | S | 10 |
| 3 | R | 4 |
| 4 | S | 10 |
| 5 | R | 6 |
| 6 | S | 10 |
| 7 | R | 8 |
| 8 | S | 10 |
| 9 | R | 10 |
| 10 | S | 10 |
| 11 | R | 2 |
| 12 | S | 10 |
| 13 | R | 4 |

(continued)

| Track No. | Solution | Amount applied ($\mu$l) |
|---|---|---|
| 14 | S | 10 |
| 15 | R | 6 |
| 16 | S | 10 |
| 17 | R | 8 |
| 18 | S | 10 |
| 19 | R | 10 |
| 20 | S | 10 |

Each track has an initial width on the plate of 4 mm; the initial distance between two tracks is 5 mm and the initial distance from one outermost track to the adjacent edge of the plate is 11 mm.

The plate is developed with ethanol : 25% aqueous ammonia 9:1 (v/v) as the eluent to a migration distance of about 6cm (under these conditions e.g. the ammonium cation of formula (I) with $R_a$, $R_c$ = hydrogen and R = 9-octadecen-1-yl migrates to an $R_f$ value of about 0.6). The plate is then immersed in the ninhydrine solution for 10 min and dried for 10 min at 100°C.

Calculation:

The areas of all developed spots are evaluated densitometrically with light of wavelength 480 nm using a TLC scanner 3 (CAMAG, Switzerland).

The areas obtained from tracks 1, 3, 5, 7 and 9 are used to obtain a first parabolically approximated calibration curve of area vs. amount of amine fluoride in $\mu$g. A second such calibration curve is obtained from tracks 11, 13, 15, 17 and 19.

The average area from sample tracks 2, 6, 10, 14 and 18 is converted to an amount [am1] amine fluoride in $\mu$g using the first calibration curve. The average area from sample tracks 4, 8, 12, 16 and 20 is similarly converted to an amount [am2] amine fluoride in $\mu$g using the second calibration curve.

The content of ammmonium cations of formula (I) I ppm, based on the oral care composition, [AM], is then obtained as

$$[AM] = \frac{1250([am1]+[am2])}{M} \times \frac{(MW-19(v+1))}{MW}$$

wherein M, [am1] and [am2] are as defined above, MW is the molecular weight of the pure amine fluoride used to prepare solution R, and v is as defined for formula (I).

Example 17: Determination of ammonium cations of formula (I) in an oral care composition of the invention

The procedure of this example is applicable to all other ammonium cations of formula (I) not falling under the definitions given in the heading of example 16. This determination is done on reverse phase HPTLC plates after staining with Berlin Blue.

Berlin Blue solution: Dissolve 4 g of potassium hexacyanoferrate(III) p.a. in 150 ml distilled water and add 350 ml of acetone p.a. Dissolve separately 7.5 g iron(III)chloride hexahydrate p.a. in 500 ml ethanol p.a. Mix immediately prior to use 40 ml of each of the two solutions and 80 ml of ethanol p.a.

A reference solution of the ammonium cation to be determined is prepared by dissolving an exactly known amount of the corresponding pure amine hydrofluoride in methanol p.a., to make a solution containing an exactly known content of the amine fluoride in the range of about 500 ppm, based on the solution. This reference solution is designated as R.

Sample solution: Accurately weigh (to within 0.1 mg) an amount M of approximately 1 g of the oral care composition in a 100 ml measuring flask and make up to volume with methanol p.a. Expose to ultrasonic radiation for about 15 minutes. This solution is designated as S.

The HPTLC plate is Silicagel 60 without fluorescence indicator, 10 x 20 cm (Merck no. 5626)

The reference solution and the sample solution are applied onto the HPTLC plate using an applicator Linomat IV (Camag, Switzerland) according to the following track scheme:

| Track No. | Solution | Amount applied ($\mu$l) |
|---|---|---|
| 1 | R | 1 |
| 2 | S | 3 |
| 3 | R | 2 |
| 4 | S | 3 |
| 5 | R | 3 |
| 6 | S | 3 |
| 7 | R | 4 |
| 8 | S | 3 |
| 9 | R | 5 |
| 10 | S | 3 |
| 11 | R | 1 |
| 12 | S | 3 |
| 13 | R | 2 |
| 14 | S | 3 |
| 15 | R | 3 |
| 16 | S | 3 |
| 17 | R | 4 |
| 18 | S | 3 |
| 19 | R | 5 |
| 20 | S | 3 |

Each track has an initial width on the plate of 4 mm; the initial distance between two tracks is 5 mm and the initial distance from one outermost track to the adjacent edge of the plate is 11 mm.

The plate is developed with n-pentanol : ethanol : diethyl ether : 25% aqueous ammonia 3:3:3:1 (v/v/v/v) as the eluent to a migration distance of about 6cm (under these conditions e.g. the ammonium cation of formula (I) with $R_a$, $R_b$, $R_c$ = 2-hydroxyethyl, R = 9-octadecen-1-yl, v = 1 and u = 3 migrates to an $R_f$ value of about 0.8). The plate is then immersed in the Berlin Blue solution for 10 min and dried for 10 min at 100°C.

Calculation:

The areas of all developed spots are evaluated densitometrically with light of wavelength 592 nm using a TLC scanner 3 (CAMAG, Switzerland).

The areas obtained from tracks 1, 3, 5, 7 and 9 are used to obtain a first parabolically approximated calibration curve of area vs. amount of amine fluoride in $\mu$g. A second such calibration curve is obtained from tracks 11, 13, 15, 17 and 19.

The average area from sample tracks 2, 6, 10, 14 and 18 is converted to an amount [am1] amine fluoride in $\mu$g using the first calibration curve. The average area from sample tracks 4, 8, 12, 16 and 20 is similarly converted to an amount [am2] amine fluoride in $\mu$g using the second calibration curve.

The content of ammmonium cations of formula (I) I ppm, based on the oral care composition, [AM], is then obtained as

$$[AM] = \frac{100000([am1]+[am2])}{6M} \times \frac{(MW-19(v+1))}{MW}$$

wherein M, [am1] and [am2] are as defined above, MW is the molecular weight of the pure amine fluoride used to prepare solution R, and v is as defined for formula (I).

Example 18: Potentiometric chloride determination in an oral care composition of the invention

A combined silver/silver chloride electrode type 6.0350.100 of Metrohm, Switzerland, and a potentiometer Titrando 809 of Metrohm, Switzerland, are used. The calibration of the electrode is done according to the manual.

1000 $\pm$ 0.1 mg of the oral care composition are exactly weighed in a 100 ml plastic container and 50 ml water and 2 ml 65 wt% nitric acid are added.

The electrode is immersed into the sample and the sample is titrated with standard 0.01 M silver nitrate solution to the endpoint of the titration. The used volume of silver nitrate solution in ml is taken as v.

The chloride contained in the sample in ppm based on the composition, [Cl⁻], is obtained as

$$[\text{Cl}^-] = 354.5\ \text{v}$$

Example 19: In situ demineralisation tests with mouthrinses of the invention

The tests were carried out on enamel and dentin samples cut from extracted third molar teeth. The erosion tests were done ex situ, using a citric acid solution, and the treatment tests were done in situ, by letting probands carry the eroded samples in their mouth using a sample holder fixed to their jaw, and by using the mouthrinses of the invention. The test was carried out as a double-blind randomized test.

The enamel and dentin samples were prepared as follows: From the teeth were removed any remaining soft tissues and the roots. From the teeth surfaces on either the enamel or the dentin part were excised samples of about 1 mm thickness in the longitudinal direction of the tooth. The face of the samples representing the original, natural tooth surface was polished to yield a flat test surface of at least 3 x 3 mm using firstly grit paper of grain size 12 $\mu$m, then of 5 $\mu$m (the dentin or enamel, respectively, was removed to a maximum depth of about 250 $\mu$m). A total of 96 enamel and of 96 dentin samples was prepared in this way. The samples were stored until the tests in a refrigerator in a thymol solution, which was freshly prepared once a week.

The probands were eight persons having good oral conditions (no artificial dentures, no open carious lesions or obviously defect dental fixtures, no visible plaque). They had teeth salivary flow rates in the ranges of 0.25-0.35 ml/min (unstimulated) and 1.0-3.0 ml/min (stimulated); salivary buffering capacities in the ranges of 4.25-4.75 (unstimulated) and 5.75-6.5 (stimulated); and salivary pH values in the ranges of 6.5-6.9 (unstimulated) and 7.0-7.5 (stimulated).

The said sample holders for jaw insertion were individually modelled for each proband and had on each side three buccal supports for either two dentin samples and one enamel sample or for one dentin sample and two enamel samples. These sample holders were thus adapted to have three enamel and three dentin samples for each proband. The sample holders were disinfected prior to use by the proband by soaking for 30 minutes in 75 vol-% aqueous ethanol.

Each proband tested the mouthrinses of examples 7 and 8, the commercially available meridol® mouthrinse mentioned in the introduction and a placebo solution devoid of both stannous ions and of fluoride. He tested each of these in a 7-day test period, and tested them in a randomized, different order unknown to him. The procedure for each 7-day test period was as follows:

A) Before the test period:

1) A 5-day "wash-out" period was done in which the probands performed normal oral hygiene.
2) Half of the abovementioned polished test surface of the enamel or dentin samples was covered with a light-curable resin (Technovit 7230 VLC, Kulzer-Exact, Wehrheim, Germany) and the other half was carefully cleaned of any impurities. The covered part of the surface served as the reference surface for the profilometric determination of the demineralisation, whereas the uncovered part served as the demineralisation test area.

B) During the test period, for each day of the test period

1) At about 8:30 a.m. a first ex situ demineralisation treatment of the enamel or dentin samples inserted into the sample holders was done in at least 200 ml of 0.05 M citric acid solution for 5 minutes, then the sample holders were rinsed with running tap water for 1 min.

2) After that demineralisation an in situ oral treatment of 30 seconds with 10 ml of one of the mouthrinses according to the invention, with the sample holder mounted on the proband's jaw; the mouthrinse was then spitted out but no rinsing with water was done.

3) Five more ex situ demineralisation treatments at about 10:00 a.m., 11:30 a.m., 1:00 p.m., 2:30 p.m. and 4:00 p.m., under the same conditions as in the first demineralisation treatment, but without subsequent treatment with the mouthrinses of the invention.

The probands carried the sample holders on their jaws except during the meals or for personal oral hygiene; for these periods they stored the sample holders in a humid chamber. Before going to bed the probands cleaned the sample holders, but not the enamel or dentin samples, with a toothbrush without using a toothpaste, then immersed the sample holder for 5 minutes in a 0.1% by weight chlorhexidine gluconate solution.

C) After the test period

The enamel and dentin samples were taken out of the sample holders. The dentin samples were treated for 36 hours at 30°C with a solution of 15 units collagenase (*Clostridium histolyticum type VII*, Sigma Aldrich, St. Louis, USA) in 150 ml of a solution containing 0.4 $H_3PO_4$, 1.5 g KCl, 1 g $NaHCO_3$ and 0.2 g $CaCl_2$ per litre, in order to completely remove the dentin's organic matrix, which is prone to disturb the outcome of the subsequent profilometry. The samples were then numbered, glued onto object slides and stored in a humid chamber until the profilometric determination of their demineralisation.

The profilometric determination of the demineralisation extent is a measurement of height difference between reference part and test part of the sample surface (see point A) of the description of the 7-day test period above). The height profiles of the samples were measured with a Perthometer S8P (Perthen Mahr, Goettingen, Germany) with a mechanical probe (FRW-750, Perthen Mahr, Goettingen, Germany) for dentin samples and with an optical probe (Rodenstock, Munich, Germany) for enamel samples. The object slides with the samples glued onto them were fixed onto the xy-table of the profilometer with a mouldable fixing mass. For each of the samples three profilometries were run. The profilometries were evaluated using a special software (Perthometer Concept 4.0, Perthen Mahr, Goettingen, Germany). With this software two heights were determined by linear regression, one of them from the height profile found on the reference part of the sample and the other one from the height profile of the test area of the sample, whereby for both height profiles a border area up to a distance of 0.2 mm from the border line between reference and test areas was disregarded. The height difference between the centre points of the two linear regression lines in micrometers, averaged from the three runs for each sample, was considered as the extent of demineralisation of that sample. As the extent of dentin demineralisation of a proband was considered the average of the said height differences from the three dentin samples he was carrying during the test; as the extent of enamel demineralisation of a proband was considered the average of the said height differences from the three enamel samples he was carrying during the test.

The obtained data were checked for sufficient normal distribution (Kolmogorov-Smirnov test). The comparison of the results of all probands for each of the tested solution was done by simple variation analysis (ANOVA) with the posthoc test according to Tukey. The following results were obtained for the four solutions tested:

| mouthrinse | extent of demineralisation on enamel (micrometres) | extent of demineralisation on dentin (micrometres) |
| --- | --- | --- |
| example 7 | $11.0 \pm 5.7$ | $26.4 \pm 11.9$ |
| example 8 | $9.7 \pm 4.1$ | $26.2 \pm 6.7$ |
| meridol® | $24.5 \pm 14.4$ | $32.8 \pm 9.6$ |
| Placebo | $54.8 \pm 8.6$ | $48.5 \pm 13.0$ |

These results show that the inventive mouthrinses have particularly on enamel an efficacy in preventing demineralisation which is about 2.5 times the efficacy of the commercial meridol® mouthrinse.

Furthermore, in none of the probands a dry mouth sensation occurred, nor any reddening of the gums or efflorescence of the proband's own teeth nor of the teeth samples were observed. In particular no significant tainting of neither the proband's own teeth nor of the samples was observed, despite the increased amounts of stannous ions as compared to the meridol® solution.

Example 20: In situ test with the mouthrinse of example 8

The mouthrinse of example 8 and a solution containing only NaF in an amount corresponding to 1000 ppm fluoride were tested and evaluated in a similar setup as described in example 19, but with 20 probands and using 180 enamel samples and 180 dentin samples. The following demineralisation results were obtained:

| | extent of demineralisation on enamel (micrometres) | extent of demineralisation on dentin (micrometres) |
|---|---|---|
| mouthrinse of example 8 | 9.2 ± 3.4 | 23.9 ± 6.4 |
| solution with 1000 NaF | 24.2 ± 9.2 | 34.1 ± 9.3 |

It can be seen that the mouthrinse of example 8, containing a combination of stannous fluoride and amine fluoride amounting to a total of 1000 ppm fluoride, is much more effective particularly on enamel than the solution containing only NaF corresponding to 1000 ppm fluoride.

Examples 21-25: Mouthrinse formulations

In the following examples "AmF" or "AmF 297/400" denotes the amine hydrofluoride OLAFLUR. The amounts of all ingredients listed in the table are in percentages by weight, based on the overall mouthrinse.

| Example No. | 21 | 22 | 23 | 24 | 25 * |
|---|---|---|---|---|---|
| Fluoride<br><br>[Sn]<br>[F⁻] | 125 ppm ex AmF,<br><br>375 ppm ex NaF<br><br>800 ppm<br>500 ppm = 0.63[Sn] | 125 ppm ex AmF,<br><br>375 ppm ex NaF<br><br>800 ppm<br>500 ppm = 0.63[Sn] | 125 ppm ex AmF,<br><br>375 ppm ex NaF<br><br>1000 ppm<br>500 ppm = 0.50[S] | 125 ppm ex AmF,<br><br>375 ppm ex NaF<br><br>1000 ppm<br>500 ppm = 0.50[S] | 125 ppm ex AmF,<br>125 ppm ex NaF<br><br>1100 ppm<br>250 ppm = 0.23[S] |
| AmF 297/400 | 0.893 | 0.893 | 0.893 | 0.893 | 0.893 |
| $SnCl_2$ | 0.157 | 0.157 | 0.196 | 0.196 | 0.216 |
| NaF | 0.0829 | 0.0829 | 0.0829 | 0.0829 | 0.02765 |
| sodium D-gluconate | 0.6 | 0.6 | 0.6 | 0.6 | 0.725 |
| sodium saccharin | 0.03 | 0.03 | 0.03 | 0.02 | 0.03 |
| glycerol water-free | 10 | 10 | 10 | 10 | 18 |
| Tego betain F50 | 0.2 | 0.2 | 0.2 | 0.2 | 0.2 |
| Cremophor RH 410 | 0.3 | 0.3 | 0.3 | 0.3 | 0.3 |
| aroma | 0.15 | 0.15 | 0.15 | 0.15 | 0.06 |
| polyvinyl pyrrolidone | | 1 | | 0.5 | 0.4 |
| HCl 20% | 0.02 | 0.02 | | | |
| KOH 20% | | | | 0.0543 | 0.14 |

(continued)

| Example No. | 21 | 22 | 23 | 24 | 25 * |
|---|---|---|---|---|---|
| demineralised water | 87.5671 | 86.5671 | 87.5481 | 87.0038 | 77.42835 |

| * Example 25 is not part of the invention as claimed |
|---|

Example 26: In vitro demineralisation test with mouthrinses of examples 21-25

The tests were carried out on enamel samples cut from extracted third molar teeth which had been stored beforehand in a saturated aqueous thymol solution. The enamel samples were prepared as follows: From the teeth were removed mechanically any remaining soft tissues and the roots. From the teeth surfaces were excised samples of about 1 mm thickness in the longitudinal direction of the tooth. The face of the samples representing the original, natural tooth surface was ground to a flat test surface of at least 3 x 3 mm; the enamel was removed to a maximum depth of about 250 $\mu$m. The flat test surface was polished using grit paper of grain size 12 $\mu$m, then of 5 $\mu$m. All grinding and polishing operations were carried out under water cooling of the sample. The polished samples were glued by means of a light-curable resin (Technovit 7230 VLC, Kulzer-Extract, Wehrheim, Germany) onto object slides. Half of the flat test surface of the samples was covered with the same light-curable resin and the other half was carefully cleaned of any impurities. A total of 72 samples was prepared in this way. The samples so prepared were stored until further use in a humid chamber at 100% relative humidity.

As the test solutions were used the mouthrinses of examples 21-25 and one negative control test solution containing 500 ppm F- ex NaF and all inactive excipients mentioned in the table of examples 21-25, but being devoid of AmF, stannous chloride and sodium D-gluconate. The pH-value of these test solutions was checked daily during the test.

For the erosive demineralisation test the 72 samples were divided into five test groups and one control group with 12 samples each, and inserted into sample holders (dyeing holders, Schott, Mainz, Germany). The test was run over a period of 10 days (2 x 5 working days), with six erosive demineralisation cycles and two application cycles of the test solutions being carried out daily. All treatments of the samples were carried out at room temperature. In between all test and application cycles and overnight between working days the samples were stored in a remineralisation solution containing 0.4 $H_3PO_4$, 1.5 g KCl, 1 g $NaHCO_3$ and 0.2 g $CaCl_2$ per litre. During the weekend the samples were stored in the abovementioned humid chamber at 100% relative humidity.

The treatment regime for each testing day was as follows:

1) A first demineralisation cycle was done for 5 minutes with the enamel samples inserted into dyeing cases (Schott, Mainz, Germany) containing 250 ml 0.05 M citric acid solution. The samples were then rinsed with tap water for 1 min.

2) The samples of the five test groups were subjected for 2 min to a treatment cycle with 250 ml of one of the mouthrinses of examples 21-25, whereas the samples of the sixth test group were subjected for 2 min to a control treatment cycle in 250 ml of the abovementioned negative control solution. After these treatment cycles the samples were rinsed for 1 min with tap water.

3) The samples were subject to four more demineralisation cycles as described under above 1) in intervals of one hour each.

4) After a further hour the samples were subject to a sixth demineralisation cycle as described under above 1, then the samples of the five test groups were subjected for 2 min to a treatment cycle with 250 ml of one of the mouthrinses of examples 21-25, whereas the samples of the sixth test group were subjected for 2 min to a control treatment cycle in 250 ml of the abovementioned negative control solution, as described under above 2).

At the end of the 10 day test the resin coat was removed from the protected areas of the test surfaces of the samples. The degree of demineralisation of each sample was determined by profilometry in the same manner as described for the enamel samples in example 19, with three determinations for each sample, and taking the average of these. The degree of demineralisation of each group was represented by the average and corresponding standard deviation from the average values of the 12 samples of that group.

The obtained data were first checked for sufficient normal distribution, then analysed by the software SPSS. The comparison of the results between groups was done by simple variation analysis with the posthoc test according to Tukey. The significance level was set to 0.05. The following results were obtained for the six solutions (one negative control; five mouthrinses from examples 21-25):

| test solution | average demineralisation in group ($\mu$m) | standard deviation of demineralisation ($\mu$m) | reduction in comparison to negative control |
|---|---|---|---|
| negative control | 82.6 | 18.2 | |
| example 21 | 25.8 | 4.4 | 68.8% |
| example 22 | 33.7 | 14.6 | 59.2% |
| example 23 | 26.9 | 7.3 | 67.4% |
| example 24 | 26.6 | 14.8 | 64.2% |
| example 25 * | 24.6 | 9.6 | 70.2% |
| * Example 25 is not part of the invention as claimed. | | | |

These results show that compositions with [Sn] of 800 to 1000 ppm, i.e. at least 750 ppm, and with [F⁻] in the range of 250 to 500 ppm, i.e. in the lower region of the range of 200 to 2000 ppm, have useful efficacity in the prevention or treatment of erosive tooth demineralisation of enamel. This is despite the presence of a organic acid (gluconic acid as the salt) acting as a stabilizing chelant (and thus as a possible activity reducing agent) for stannous ion.

**Claims**

1.  An oral care composition comprising a liquid phase containing 30% to 90%, preferably 30% to 80% by weight, based on the liquid phase, of water; dissolved tin; 200 to 2000 ppm fluoride ions, based on the oral composition; and 5 to 60% by weight, based on the oral care composition, of a $C_{(3-5)}$ sugar alcohol; wherein the content of dissolved tin [Sn] in the liquid phase is at least 750 ppm, preferably at least 1000 ppm, based on the composition; that 60 mol% or more, preferably 75 mol% or more of the content of dissolved tin [Sn] is in the formal oxidation state +II; and that the composition comprises an organic acid and ammonium cations of the formula (I):

$$R-NH^+R_a-[(CH_2)_u-NH^+R_b]-R_c \cdot \qquad (I)$$

wherein R is a saturated or unsaturated straight-chain hydrocarbon residue of 10 to 20 carbon atoms, v is an integer from 0 to 1, u is an integer from 2 to 3 and $R_a$, $R_b$ and $R_c$ are independently selected from hydrogen and $-CH_2CH_2OH$,
wherein the organic acid is a carboxylic acid, the content of organic acid is 0.01 to 10 % by weight, based on the composition and the content of ammonium cations of formula (I) is in the range of 1500 ppm to 10000 ppm, based on the composition,
wherein [F⁻] is in the range of 0.70 [Sn] $\geq$ [F⁻] $\geq$ 0.40 [Sn], and
wherein the composition is a mouthrinse.

2.  The composition of claim 1, **characterised in that** the total content of dissolved tin [Sn] in the liquid phase is in the range of 1000 ppm to 3000 ppm, based on the composition, optionally in the range of 1300 ppm to 2500 ppm.

3.  The composition of one of claims 1 to 2, **characterised in that** in formula (I) R is $C_{(16-20)}$alkyl or $C_{(16-20)}$alkenyl, optionally $C_{18}$alkyl or $C_{18}$alkenyl.

4.  The composition of one of claims 1 to 3, **characterised in that** in formula (I) either

    i) v is 0 and $R_a$, $R_c$ are hydrogen;
    ii) v is 0 and $R_a$, $R_c$ are $-CH_2CH_2OH$; or
    iii) v is 1, u is 3, and $R_a$, $R_b$, $R_c$ are $-CH_2CH_2OH$.

**5.** The composition of one of claims 1 to 4, **characterised in that** [F$^-$] is in the range of 0.60[Sn] $\geq$ [F$^-$] $\geq$ 0.40[Sn].

**6.** The composition of one of claims 1 to 5, for use in the treatment or prevention of erosive tooth demineralisation caused by food acids or endogeneous acids.

**7.** The composition of claim 6, wherein the use is for the prevention of erosive tooth demineralisation.

**8.** The composition of claim 6 or 7, wherein the food acids are acids with a first pKa value of 3.0 or less and/or are acids with chelating ability for calcium ions and/or which form low-soluble calcium salts, or the endogeneous acid is gastric juice.

**9.** The oral care composition according to one of claims 1 to 8 for use in a method of treating or preventing erosive tooth demineralisation caused by food acids or endogeneous acids in a subject in need of such treatment or prevention, wherein the subject's teeth are brought in contact with the oral care composition in an amount which is effective to treat or prevent such erosive tooth demineralisation, optionally wherein the subject's teeth are brought in contact with the composition for a period of time in the range of 10 seconds to 1 minute.

**10.** The oral care composition of claim 9, which is for use in preventing erosive tooth demineralisation.

**Patentansprüche**

**1.** Mundpflegezusammensetzung, die Folgendes umfasst: eine flüssige Phase, die 30 Gewichts-% bis 90 Gewichts-%, bevorzugt 30 Gewichts-% bis 80 Gewichts-%, bezogen auf die flüssige Phase, an Wasser enthält; gelöstes Zinn; 200 bis 2000 ppm Fluoridionen, bezogen auf die orale Zusammensetzung; und 5 bis 60 Gewichts-%, bezogen auf die Mundpflegezusammensetzung, eines C$_{(3-5)}$-Zuckeralkohols, wobei der Gehalt an gelöstem Zinn [Sn] in der flüssigen Phase mindestens 750 ppm, bevorzugt mindestens 1000 ppm, bezogen auf die Zusammensetzung, beträgt; dass 60 mol-% oder mehr, bevorzugt 75 mol-% oder mehr des Gehalts an gelöstem Zinn [Sn] in dem formalen Oxidationszustand +II vorliegen; und dass die Zusammensetzung eine organische Säure und Ammoniumkationen der Formel (I) umfasst:

$$R\text{-}NH^+R_a\text{-}[(CH_2)_u\text{-}NH^+R_b]_v\text{-}R_c \qquad (I),$$

wobei R ein gesättigter oder ungesättigter geradkettiger Kohlenwasserstoffrest von 10 bis 20 Kohlenstoffatomen ist, v eine ganze Zahl von 0 bis 1 ist, u eine ganze Zahl von 2 bis 3 ist und $R_a$, $R_b$ und $R_c$ unabhängig aus Wasserstoff und -CH$_2$CH$_2$OH ausgewählt sind,
wobei die organische Säure eine Carbonsäure ist, der Gehalt an organischer Säure 0,01 bis 10 Gewichts-%, bezogen auf die Zusammensetzung, beträgt und der Gehalt an Ammoniumkationen der Formel (I) im Bereich von 1500 ppm bis 10000 ppm, bezogen auf die Zusammensetzung, liegt,
wobei [F$^-$] im Bereich von 0,70 [Sn] $\geq$ [F$^-$] $\geq$ 0,40 [Sn] liegt und
wobei die Zusammensetzung eine Mundspülung ist.

**2.** Zusammensetzung nach Anspruch 1, **dadurch gekennzeichnet, dass** der Gesamtgehalt an gelöstem Zinn [Sn] in der flüssigen Phase im Bereich von 1000 ppm bis 3000 ppm, bezogen auf die Zusammensetzung, optional im Bereich von 1300 ppm bis 2500 ppm liegt.

**3.** Zusammensetzung nach einem der Ansprüche 1 bis 2, **dadurch gekennzeichnet, dass** in Formel (I) R C$_{(16-20)}$-Alkyl oder C$_{(16-20)}$-Alkenyl, optional C$_{18}$-Alkyl oder C$_{18}$-Alkenyl ist.

**4.** Zusammensetzung nach einem der Ansprüche 1 bis 3, **dadurch gekennzeichnet, dass** in Formel (I) entweder

i) v 0 ist und $R_a$, $R_c$ Wasserstoff sind;
ii) v 0 ist und $R_a$, $R_c$ -CH$_2$CH$_2$OH sind; oder
iii) v 1 ist, u 3 ist und $R_a$, $R_b$, $R_c$ -CH$_2$CH$_2$OH sind.

**5.** Zusammensetzung nach einem der Ansprüche 1 bis 4, **dadurch gekennzeichnet, dass** [F$^-$] im Bereich von 0,60 [Sn] $\geq$ [F$^-$] $\geq$ 0,40 [Sn] liegt.

6. Zusammensetzung nach einem der Ansprüche 1 bis 5 für die Verwendung bei der Behandlung oder Prävention von erosiver Zahndemineralisation, die durch Lebensmittelsäuren oder endogene Säuren verursacht wird.

7. Zusammensetzung nach Anspruch 6, wobei die Verwendung für die Prävention von erosiver Zahndemineralisation ist.

8. Zusammensetzung nach Anspruch 6 oder 7, wobei die Lebensmittelsäuren Säuren mit einem ersten pKa-Wert von 3,0 oder weniger sind und/oder Säuren mit Chelatisierungsfähigkeit für Calciumionen und/oder solche sind, die gering lösliche Calciumsalze bilden, oder die endogene Säure Magensaft ist.

9. Mundpflegezusammensetzung nach einem der Ansprüche 1 bis 8 für die Verwendung in einem Verfahren zur Behandlung oder Prävention von erosiver Zahndemineralisation, die durch Lebensmittelsäuren oder endogene Säuren verursacht wird, bei einem Subjekt, das eine derartige Behandlung oder Prävention benötigt, wobei die Zähne des Subjekts mit der Mundpflegezusammensetzung in einer Menge in Kontakt gebracht werden, die für die Behandlung oder Prävention von derartiger erosiver Zahndemineralisation wirksam ist, optional wobei die Zähne des Subjekts mit der Zusammensetzung für eine Zeitdauer im Bereich von 10 Sekunden bis 1 Minute in Kontakt gebracht werden.

10. Mundpflegezusammensetzung nach Anspruch 9, die für die Verwendung bei der Prävention von erosiver Zahndemineralisation ist.

## Revendications

1. Composition de soins buccaux comprenant une phase liquide contenant 30 % à 90 %, préférablement 30 % à 80 % en poids d'eau, d'étain dissout, par rapport à la proportion de la phase liquide ; 200 à 2000 ppm d'ions de fluorure, par rapport à la proportion de la composition de soins buccaux ; et 5 à 60 % en poids d'un alcool de sucre en $C_3$ à $C_5$ par rapport à la proportion de la composition de soins buccaux ; dans laquelle la teneur en étain dissout [Sn] dans la phase liquide est d'au moins 750 ppm, préférablement d'au moins 1000 ppm, par rapport à la proportion de la composition ; basé sur le fait que 60 % en moles ou plus, préférablement 75 % en moles ou plus de la teneur en étain dissout [Sn] sont à l'état d'oxydation formel +II ; et basé sur le fait que la composition comprend un acide organique et des cations d'ammonium de la formule (I) :

$$R\text{-}NH^+R_a\text{-}[(CH_2)_u\text{-}NH^+R_b]_v\text{-}Rc \cdot \qquad (I)$$

où R représente un résidu d'hydrocarbure à chaîne droite saturé ou insaturé portant 10 à 20 atomes de carbone, v désigne un nombre entier valant de 0 à 1, u désigne un nombre entier valant de 2 à 3 et $R_a$, $R_b$ et $R_c$ sont indépendamment sélectionnés parmi l'hydrogène et -$CH_2CH_2OH$,
dans laquelle l'acide organique est un acide carboxylique, la teneur en acide organique est de 0,01 à 10 % en poids, par rapport à la proportion de la composition et la teneur en cations d'ammonium de la formule (I) est comprise dans l'intervalle de 1500 ppm à 10 000 ppm, par rapport à la proportion de la composition,
dans laquelle [F-] est compris dans l'intervalle de 0,70 [Sn] $\geq$ [F-] $\geq$ 0,40 [Sn], et
dans laquelle la composition est un rince-bouche.

2. Composition selon la revendication 1, **caractérisée en ce que** la teneur totale en étain dissous [Sn] dans la phase liquide est comprise dans l'intervalle de 1000 ppm à 3000 ppm, par rapport à la proportion de la composition, éventuellement dans la plage de 1300 ppm à 2500 ppm.

3. Composition selon l'une quelconque des revendications 1 à 2, **caractérisée en ce que** dans la formule (I) R représente un alkyle en $C_{16}$ à $C_{20}$ ou un alcényle en $C_{16}$ à $C_{20}$, éventuellement un alkyle en $C_{18}$ ou un alcényle en $C_{18}$.

4. Composition selon l'une des revendications 1 à 3, **caractérisée en ce que** dans la formule (I) soit

i) v désigne 0 et $R_a$, $R_c$ représentent l'hydrogène ;
ii) v désigne 0 et $R_a$, $R_c$ représentent -$CH_2CH_2OH$ ; ou
iii) v vaut 1, u vaut 3 et $R_a$, $R_b$, $R_c$ représentent -$CH_2CH_2OH$.

5. Composition selon l'une des revendications 1 à 4, **caractérisée en ce que** [F-] est compris dans l'intervalle de 0,60

$[Sn] \geq [F^-] \geq 0{,}40\ [Sn]$.

6. Composition selon l'une des revendications 1 à 5, pour utilisation dans le traitement ou la prévention de la déminéralisation érosive des dents causée par des acides alimentaires ou des acides endogènes.

7. Composition selon la revendication 6, dans laquelle l'utilisation destinée à la prévention de la déminéralisation érosive des dents.

8. Composition selon la revendication 6 ou la revendication 7, dans laquelle les acides alimentaires sont des acides ayant une première valeur pKa de 3,0 ou inférieure et/ou sont des acides ayant une capacité de chélation pour les ions de calcium et/ou qui forment des sels de calcium faiblement solubles, ou l'acide endogène est du jus gastrique.

9. Composition de soins buccaux selon l'une quelconque des revendications 1 à 8 pour une utilisation dans un procédé de traitement ou de prévention de la déminéralisation érosive des dents causée par des acides alimentaires ou des acides endogènes chez un sujet ayant besoin d'un tel traitement ou d'une telle prévention, dans laquelle les dents du sujet sont mises en contact avec la composition de soins buccaux en une quantité qui est efficace pour traiter ou prévenir une telle déminéralisation érosive des dents, éventuellement dans laquelle les dents du sujets sont mises en contact avec la composition pendant une période de temps comprise dans l'intervalle de 10 secondes à 1 minute.

10. Composition de soins buccaux selon la revendication 9, qui est destinée à une utilisation dans la prévention de la déminéralisation érosive des dents.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 08155126 A
- EP 09153802 A
- US 5004597 A
- EP 0026539 A
- WO 9822427 A

**Non-patent literature cited in the description**

- *Archs. Oral Biol.,* 1971, vol. 16, 241ff
- *J. Dent. Res.,* 1971, vol. 50/3, 531ff
- *Caries Res.,* 2008, vol. 42, 2-7